# EUROPEAN PATENT APPLICATION

(11) **EP 1 419 771 A1**
(43) Date of publication of application: **19.05.2004**
(21) Application number: 02762812.2
(22) Date of filing: 21.08.2002
(51) Int. Cl.: A61K 31/426, A61P 9/00, A61P 9/10, A61P 43/00, C07D 277/34

(54) **PREVENTIVE AND/OR REMEDIAL AGENT FOR DISEASE ATTRIBUTABLE TO ARTERIOSCLEROTIC ACTIVITY**

(30) Priority: 23.08.2001 JP 2001252388
(71) Applicant: Mitsubishi Pharma Corporation, Osaka-shi, Osaka 541-0046 (JP)
(72) Inventor: ISHII, S., MITSUBISHI PHARMA CORP., Head Office, Tokyo 103-8405 (JP); SASAKI, K., MITSUBISHI PHARMA CORP., Head Office, Tokyo 103-8405 (JP); UENO, H., MITSUBISHI PHARMA CORP., Head Office, Tokyo 103-8405 (JP)
(74) Representative: von Kreisler, Alek, Dipl.-Chem.
(86) International application number: PCT/JP2002/008398
(87) International publication number: WO 2003/018010

(57) **Abstract**

A preventive and/or therapeutic medicament for diseases attributed to arteriosclerotic activity such as ischemic heart diseases and acute coronary arterial syndromes and the like, which contains as an active ingredient a compound represented by the following formula (I) or a pharmaceutically acceptable salt thereof:

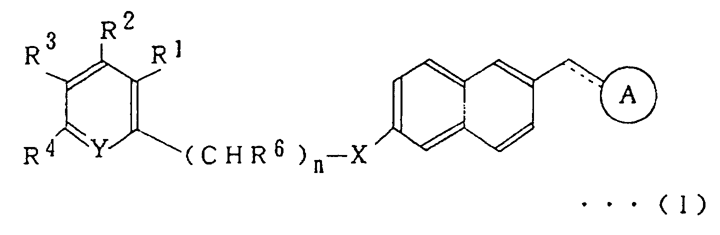

A preventive and/or therapeutic agent for diseases based on arteriosclerotic activity which is more potent than known drugs hereinbefore and novel is provided.

In the formula, A represents a thiazolidinedione ring and the like; -X- represents -O- or -S-; =Y- represents =N- or =CR⁵⁻; R¹ R², R³, R⁴ and R⁵ each independently represent hydrogen and the like; n is an integer of 0 to 3; and the dotted line indicates that said linkage may be a double bond.

## Description

### TECHNICAL FIELD

This invention relates to a preventive and/or therapeutic medicament for diseases attributed to arteriosclerotic activity, and in more detail, to a preventive and/or therapeutic medicament for diseases attributed to arteriosclerotic activity such as ischemic heart diseases and acute coronary arterial syndrome containing as the active ingredient specific naphthalene derivatives.

### BACKGROUND ART

The present invention provides a novel preventive and/or therapeutic medicament for the diseases attributed to arteriosclerotic activity.

As PPAR (Peroxisome Proliferator-activated Receptor), there are known three subtypes, PPARα, PPARγ, and PPARδ. As activators of PPARα, PPARγ, and PPARδ, fibrate-type anti-hyperlipemia agents, thiazolidinedione-type insulin sensitizers, and a PPARδ selective agonist GW501516 are known, respectively.

It is known that activation of PPARα causes an improving effect on lipid metabolism as well as the preventing and/or treating effects on arteriosclerosis such as ischemic heart diseases or cerebrovascular disorders. Specifically, it has been reported that the fibrate-type agents which have been reported to have an anti-arteriosclerotic effect in humans, improve lipid metabolism due to the increasing β-oxidation in the liver accompanied with PPARα activation, as well as an increasing high density lipoprotein (HDL) levels in blood due to an increasing ApoA-I production, a suppressing effect on the expression of cell adhesion molecules or endothelin-1 in vascular endothelial cells, an anti-inflammatory effect such as the suppressed production of inflammatory cytokines in vascular smooth muscles, a suppressing effect on the expression of Tissue Factor in monocytes or macrophages, and an activation of reverse cholesterol transport system.

It has been reported based on in vitro experimental results that activation of PPARγ causes the lowering effect on blood glucose and lipid levels due to an increased insulin sensitivity, as well as the inhibitory effect on the growth of vascular smooth muscles, the suppressing effect on migration of vascular smooth muscles due to suppression of MMP production, the inhibitory effect on the expression of adhesion molecules such as VCAM-1 or ICAM-1 in monocytes or macrophages, the suppressing effect on the production of inflammatory cytokines such as TNF-α, IL-1β, or IL-6 from macrophages, and the suppressing effect on the production of MMP-9 (Diabetes Care, 2001, 24:392). It is conceivable that these effects on blood vessels are anti-arteriosclerotic effects. In fact, recent reports show the anti-arteriosclerotic effect of Troglitazone as a PPARγ agonist on LDL receptor knockout mice or ApoE knockout mice (J.Clin.Invest. 2000, 106:523, Artherioscler. Thromb. Vasc. Biol. 2001.21:365, Artherioscler. Thromb. Vasc. Biol. 2001.21:372).

Although there is a lot of uncertainty about the function of PPARδ, the results of an experiment using L-165041 or GW501516 as a PPARδ agonist suggest that PPARδ is involved in cholesterol metabolism. In fact, it has been reported that activation of PPARδ increases HDL and ApoA-1 in blood and promotes the reverse cholesterol transport system due to increased expression of ATP-binding cassette A1 (ABC-A1) (Proc. Natl. Acad. Sci. USA 2001, 98:5306).

The international publication gazette WO 98/05331 describes that a combination therapy comprising a PPARα agonist and a PPARγ agonist is more useful in treating diabetes and arteriosclerosis as compared with a single administration of a PPARα agonist or a PPARγ agonist. Further, in the international publication gazette WO 96/01317, although the importance of effects through PPARγ or PPARδ on arteriosclerosis is suggested, there is no description about an agent having the effect of activating PPARα, PPARγ, and PPARδ simultaneously. In addition, there is no description and suggestion about the use of an agent containing a compound having the effect of activating PPARα, PPARγ, and PPARδ simultaneously as an agent for preventing and/or treating arteriosclerosis.

On the other hand, Japanese Patent Unexamined Publication (Kokai) No. Hei 6-247945 describes that a compound which is an active ingredient in the present invention can be used as a more potent agent for treating diabetes having few side effects. However, the relationship between the compound and arteriosclerosis has not been reported at all until now so far as the present inventors know. Also, the effect of activating PPARδ and the effect of activating all of PPARα, PPARγ, and PPARδ have not been reported at all until now so far as the present inventors know. Further, the aforementioned Japanese Patent Unexamined Publication (Kokai) does not describe at all that the compound which is an active ingredient in the present invention has the effect of suppressing the expression of adhesion molecules in vascular endothelial cells and the effect of suppressing the secretion of molecules causing adhesion and migration of monocytes in vascular endothelial cells, and such a report has not been confirmed at all so far as the present inventors know.

The present invention provides to a novel preventive and/or therapeutic medicament for diseases attributed to arteriosclerotic activity,

### DISCLOSURE OF THE INVENTION

The present inventors have found that the compounds represented by the following formula (I) exhibit simultaneously PPARα, γ, and δ, and are promising as the preventive and/or therapeutic medicament for the diseases attributed to arteriosclerotic activity, and have completed the present invention.

Namely, the gist of the present lies in the preventive and/or therapeutic medicament for the diseases attributed to arteriosclerotic activity which contains as the active ingredient the compounds of the following formula (I) or pharmaceutically acceptable salts thereof: 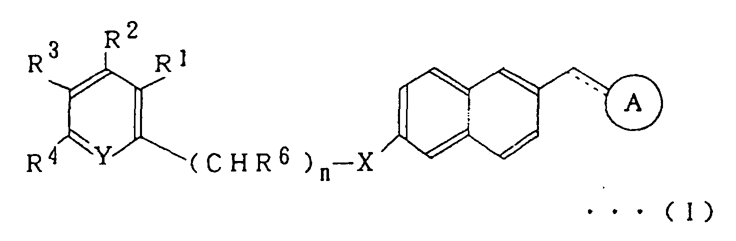 In the above formula, 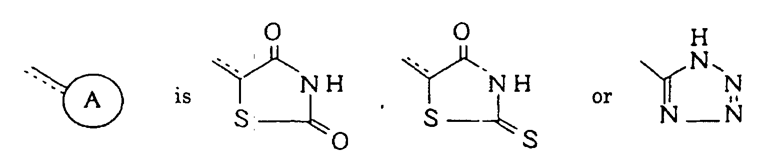 -X- represents -O- or -S-; =Y- represents =N- or =CR⁵⁻; wherein R¹, R², R³, R⁴ and R⁵ each independently represents hydrogen atom, a halogen atom, an alkyl group, an aryl group, an alkoxy group, an alkoxyalkoxy group, an aryloxy group, alkanoyloxy group, an arylcarbonyloxy group, carboxyl group, an alkoxycarbonyl group, an aryloxycarbonyl group, carbamoyl group, an alkylaminocarbonyl group, an arylaminocarbonyl group, amino group, an alkylamino group, an alkanoylamino group, an arylcarbonylamino group, ethylenedioxymethyl group, formyl group, cyano group, nitro group or a trihalomethyl group; R⁶ represents hydrogen atom, an alkyl group which may be substituted or an aryl group which may be substituted; n is an integer of 0 to 3; and the dotted line indicates that the linkage may be a double bond.

Further, preferable embodiment of the present invention includes the aforementioned preventive and/or therapeutic medicament attributed to arteriosclerotic activity wherein 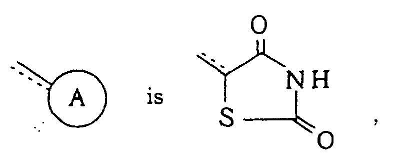 -X- represents -O-; =Y- represents =CR⁵-; R¹, R², R³ and R⁴ each independently represents hydrogen atom or a halogen atom; R⁵ represents hydrogen atom; R⁶ represents hydrogen atom; n is 1; and the dotted line indicates that said linkage is a single bond, in particular, the aforementioned preventive and/or therapeutic medicament attributed to arteriosclerotic activity wherein R¹ represents fluorine atom; R², R³ and R⁴ each represents hydrogen atom. The diseases attributed to arteriosclerotic activity include preferably ischemic heart diseases and acute coronary arterial syndrome.

The second gist of the present invention includes a PPARδ activating agent which comprises as the active ingredient the aforementioned compounds of formula (I) or pharmaceutically acceptable salts thereof, and preferable embodiment includes the PPARδ activating agent wherein 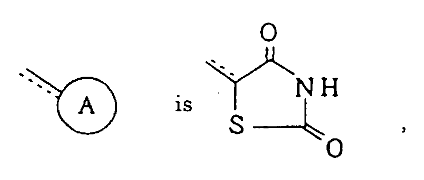 -X- represents -O-; =Y- represents =CR⁵-; R¹, R², R³ and R⁴ each independently represents hydrogen atom or a halogen atom; R⁵ represents hydrogen atom; R⁶ represents hydrogen atom; n is 1; and the dotted line indicates that said linkage is a single bond, in particular, more preferable embodiment includes that R¹ represents fluorine atom; R², R³ and R⁴ each represents hydrogen atom. Further, preferable embodiment includes the medicament having not only PPARδ activating effect, but also PPARα and PPARγ activating effects.

The third gist of the present invention includes an inhibitor for the expression of the adhesion molecule in the vascular endothelial cell which contains as the active ingredient the aforementioned compounds of the formula (I) or pharmaceutically acceptable salts, the preferable embodiment includes the PPARδ activator wherein 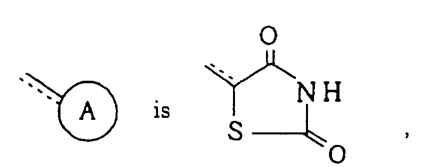 -X- represents -O-; =Y- represents =CR⁵⁻; R¹, R², R³ and R⁴ each independently represents hydrogen atom or a halogen atom; R⁵ represents hydrogen atom; R⁶ represents hydrogen atom; n is 1; and the dotted line indicates that said linkage is a single bond, in particular, more preferable embodiment includes that R¹ represents fluorine atom; R², R³ and R⁴ each represents hydrogen atom. Further, preferable adhesion molecule preferably includes VCAM-1.

The fourth gist of the present invention includes an inhibitor for the secretion of the molecule caused the adhesion and/or migration of monocytes in the vascular endothelial cell which contains the aforementioned compounds of the formula (I) or pharmaceutically acceptable salts thereof, the preferable embodiment includes the PPARδ activator wherein 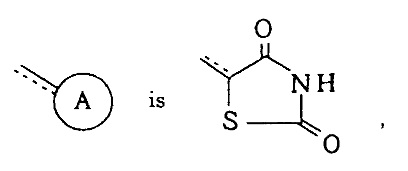 -X- represents -O-; =Y- represents =CR⁵-; R¹, R², R³ and R⁴ each independently represents hydrogen atom or a halogen atom; R⁵ represents hydrogen atom; R⁶ represents hydrogen atom; n is 1; and the dotted line indicates that said linkage is a single bond, in particular, more preferable embodiment includes that R¹ represents fluorine atom; R², R³ and R⁴ each represents hydrogen atom. Further, the preferable embodiment of the molecule caused the adhesion and/or migration of monocytes includes MCP-1.

### BRIEF DESCRIPTION OF THE INVENTION

Fig. 1 shows the effects on the expression of the adhesion molecule in the vascular endothelial cell.
Fig. 2 shows the effects on the secretion of MCP-1 from the vascular endothelial cell.

### BEST MODE FOR CARRYING OUT THE INVENTION

The followings are the detailed explanation of the present invention and the compounds as the active ingredient of the present invention are the naphthalene compounds as described in the aforementioned formula (I) or pharmaceutically acceptable salts thereof. The examples of the compounds as described in the aforementioned formula (I) include the compounds described in Japanese Patent Unexamined Publication (Kokai) No. Hei 6-247945. The preferable compounds in the compounds of the present invention include the compounds in the aforementioned formula (I) wherein 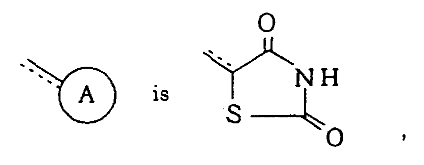 X represents -O-; =Y- represents =CR⁵-; R¹, R², R³ and R⁴ each independently represents hydrogen atom or a halogen atom; R⁵ represents hydrogen atom; R⁶ represents hydrogen atom; n is 1; and the dotted line indicates that said linkage is a single bond, and particularly preferable compounds are R¹ represents fluorine atom; and R², R³ and R⁴ each independently represents hydrogen atom. The salts of these compounds include salts with non-toxic bases, and preferable salts include salts with inorganic bases such as sodium salt, potassium salt and the like, ammonium salt or salts with organic bases such as triethylamine and the like.

The compounds as the active ingredient of the present invention embrace the compounds having an asymmetric carbon atom, and in such case the isolated stereoisomer or mixture thereof also embraced in the present invention. Further, the crystal polymorphs described in the international publication gazette WO 2000/31055 and WO 2001/36401 can be used as the active ingredient of the present invention.

The compounds of the present invention are known compounds, and foe example, can be easily prepared according to the methods in the Japanese Patent Unexamined Publication (Kokai) No. Hei 6-247945, the international publication gazette WO 2000/31055 and WO 2001/36401, or a similar methods thereto.

The aforementioned compounds exhibit the PPARα, PPARγ, and PPARδ activating activities and can be used as the preventive and/or therapeutic medicaments for diseases attributed to arteriosclerotic activity. The diseases attributed to arteriosclerotic activity include, for example, ischemic heart diseases, acute coronary arterial syndromes (ACS) and the like.

The aforementioned compounds have the activating activity for all of PPARα, PPARγ, and PPARδ, the suppressing activity to the expression of VCAM-1, the adhesion molecule in the vascular endothelial cells, as well as the suppressing activity to the secretion of MCP-1, molecule for inducing the adhesion and migration of monocytes in the vascular endothelial cells. Consequently, the compounds of the present invention are effective as more potent preventive and/or therapeutic medicaments for diseases attributed to the arteriosclerotic activity compared with the conventional medicaments.

The aforementioned compounds can be prepared in suitable formulations to the administration route with conventional carriers. For example, they can be formulated into tablets, capsules, granules, powders, liquids and the like for oral administration. In preparing a solid formulation for oral administration, conventional excipients, binders, lublicants, other coloring agents, disintegrators and the like can be used.

The excipients include, for example, lactose, starch, talc, magnesium stearate , crystal cellulose, methylcellulose, carboxylmethylcellulose, glycerin, sodium arginate, arabic gum and the like. The binders include polyvinyl alcohol, polyvinyl ether, ethylcellulose, arabic gum, shellac, sucrose and the like. The lublicants include magnesium stearate, talc and the like. The other conventional coloring agents and disintegrators can also be used.

Further, the liquid formulations are preferably selected from aqueous or oily suspensions, solutions, syrups, elixirs and others and prepared according to the conventional methods. In preparing injection, pH adjusting agents, buffers, stabilizers, isotonic agents, local anesthetics and the like to added the aforementioned compounds and the subcutaneous, intramuscular, and intraveneous injection can be prepared by the conventional manner

Bases for preparing suppositories include, for example, oil and fat bases such as cacao butter, polyethyleneglycol, Witepzol (registered trademark, Dynamite Nobel Corp.).

The dosage of the medicaments thus prepared depends on the symptoms, body weights, ages and the like of the patients and then the medicaments cannot be administered in the same manner. The amount ranging about 0.01 to 200 mg of the aforementioned compounds per day for the adults is generally preferable and the patients preferably administered once to four times-divided form a day.

### EXAMPLE

The present invention will be explained according to the examples in more detail. However, the present invention is not limited to these examples as far as not exceeded over the gist of the present invention.

### Example 1

The effect of activating PPARα, PPARγ, and PPARδ of an A type crystal of 5-[6-(2-fluorobenzyloxy)-2-naphthyl]-methyl-thiazolidine-2,4-dione (hereinafter also referred to as "MCC-555") obtained according to a method described in the international publication gazette WO 2000/31055 was investigated as follows.

An effect on the transcription activity of PPARα, PPARγ, and PPARδ was investigated using 293T cells into which there were introduced a vector obtained by fusing the ligand-binding domain of human PPARα, PPARγ, or PPARδ and the DNA-binding domain of GAL4 (hereinafter also abbreviated as a "Gal4-hPPARα (LBD) vector", a "Gal4-hPPARγ (LBD) vector", or a "Gal4-hPPARδ (LBD) vector"), and a reporter gene plasmid containing a luciferase gene placed downstream from a GAL4 responsive element (hereinafter also abbreviated as "Gal4-Luc"). The 293T cells were prepared by introducing T antigens into 293 cells (ATCC, CRL-1573) according to a method by DuBridge, et al (Mol.Cell.Boil., 1987, vol 7, 379-387).

In usual, the 293T cells are cultured in DMEM (Sigma) containing 10% FBS (Gibco BRL) in a CO₂ incubator (5% CO₂, 37°C). In a case where the 293T cells are used for a study, they are cultured in DMEM containing 10% of delipidated FBS treated with charcoal and an ion-exchange resin AG1-X8 Resin (BioRad) (hereinafter also abbreviated as "DMEM (+)").

On the first day, the 293T cells were cultured in 6-well plates at a density of 1 × 10⁵ cells/well with DMEM (+). On the second day, a transfection mixture, which contained with 9 µL of TransIT-LT1 (Takara), 2 µg of the Gal4-hPPARα (LBD) vector, the Gal4-hPPARγ (LBD) vector or the Gal4-hPPARδ (LBD) vector, 1 µg of the Gal4-Luc and 200 µL of DMEM (without FBS), was gently added to the cells at 200 µL per well. The cells were cultured all day and night to carry out gene introduction to the cells. For the investigation of the specificity of the effect on the introduced PPAR genes, gene introduction was carried out in the same manner described above using a vector without PPAR ligand-binding domain (hereinafter abbreviated as a "Gal4-control vector"). On the third day, the cells in 3 wells of the 6-well plates were combined to adjust the concentration of cells to 3 × 10⁵ cells/mL, and the obtained one was dispensed in 96-well plates at 100 µL/well to culture the cells. On the fourth day, the culture medium was replaced with 50 µL of the DMEM (+) containing the test compound at various concentrations (0.03 to 30 µM) (final DMSO concentration: 0.1%) to culture the cells. After the cells were exposed to the compound for 32 hours, 50 µL of Luc-Screen (Applied Biosystems) was added. 70 µL of the reaction solution in each well was moved to white plates to measure luminescence emitted due to the reaction of luciferase by the use of a Microplate Luminometer (EG & G berthold, LB96P). The obtained luminescence intensity was used as an index of the production quantity of luciferase.

The specific activities of the luminescence intensity of a compound addition group to a control group (DMSO 0.1%) were determined, and EC₅₀ values and 95% confidence intervals were calculated from dose-response curves.

The results are shown in the following table. Also, data of known compounds having a similar structure (Pioglitazone represented by the following formula (II) and Rosiglitazone represented by the following formula (III)) are shown.

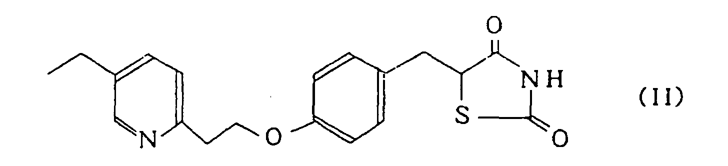

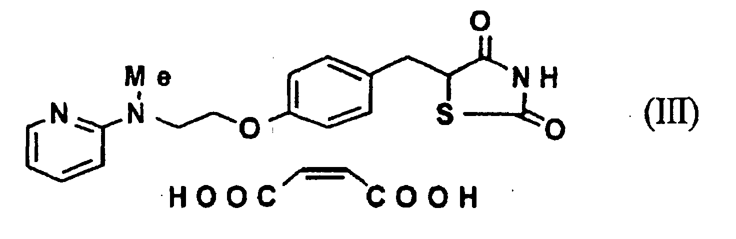

**Table**

| EC₅₀ values of PPARs transcription activation effect (95% confidence interval: µM) | | | |
|---|---|---|---|
| Compound | PPARα | PPARγ | PPARδ |
| MCC-555 | 1.0 (0.8-1.3) | 6.2 (5.0-7.6) | 1.2 (0.9-1.5) |
| Pioghtazone | 12.7 (6.6-24.6) | 7 (2.0-3.8) | Activity was not confirmed |
| Rosiglitazone | 24.2 (1.0-572.1) | 4 (0.3-0.7) | 10.8 (7.4-15.7) |

As was apparent from the above results, the compounds of the present invention have EC₅₀ values at the same level as Pioglitazone and Rosiglitazone known as PPARγ agonists, and have. the effect of activating all of PPARα, PPARγ, and PPARδ.

Therefore, it is inferred from the results that the compounds of the present invention are more effective as a potent medicament for preventing and/or treating arteriosclerosis as compared with conventional agents.

### Example 2

An effect on the expression of VCAM-1 (Vascular Cell Adhesion Molecule-1), an adhesion molecule in vascular endothelial cells was investigated using the MCC-555 mentioned in Example 1 as follows.

An effect on the expression of adhesion molecules in vascular endothelial cells was investigated using human aortic endothelial cells (available from Clonetics Corp., USA, and hereinafter abbreviated as "HAEC"). In usual, the HAEC cells were cultured in EGM-2 medium (Clonetics) containing 2% FBS (Clonetics) in a CO₂ incubator (5% CO₂, 37°C). On the first day, the HAEC cells suspended in EGM-2 medium containing 2% FBS were seeded in 96-well plates at 1 × 10⁵ cells/well and then cultured. On the second day, after the cells were washed with PBS, the culture medium was replaced with 200 µL of EGM-2 medium containing 0.4% FBS and the test agent at various concentrations (0.03 to 30 µM) (final DMSO concentration: 0.1%) to culture the cells for 24 hours. On the third day, after the cells were washed with PBS, the culture medium was replaced with 200 µL of EGM-2 medium containing 0.4% FBS, TNF-α (10 ng/mL) and the test agent at various concentrations (0.03 to 30 µM) (final DMSO concentration: 0.1%) to stimulate the cells for 4 hours. After stimulation with TNF-α, the cells were washed with PBS, and then the amount of VCAM-1 expressed was evaluated according to the following cell ELISA method using an anti-human VCAM-1 antibody solution (PharMingen). Namely, the cells were fixed and blocked using paraformaldehyde, and then 200 µL of the anti-human VCAM-1 antibody solution (PharMingen) was added as a primary antibody to induce a primary antibody response for overnight incubation. Next day, 200 µL of HRP-labeled anti-Mouse IgG (γ + L) Goat F (Ab7)2 was added as a secondary antibody to induce a secondary antibody response for 4 hours, and then a substrate solution (ortho-phenylenediamine and hydrogen peroxide) was added. After reaction, the absorbance was measured using a microplate spectrophotometer, and the obtained absorbance was used as an index of the amount of VCAM-1 expressed.

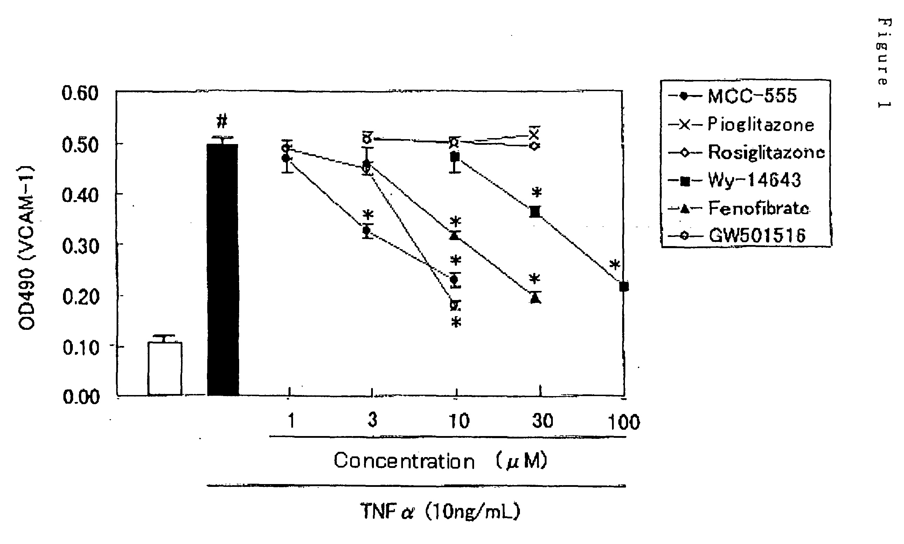

The results are shown in FIG. 1. Also, data of known compounds having a similar structure (Pioglitazone represented by the formula (II) and Rosiglitazone represented by the formula (III)) are shown.

As was apparent from the results, although Pioglitazone and Rosiglitazone known as PPARγ agonists had no effect on the expression of VCAM-1 induced by stimulation with TNF-α, the compounds of the present invention suppressed the expression of VCAM-1 induced by stimulation with TNF-α in human vascular endothelial cells as the same case with Fenofibrate and Wy-14643 known as PPARα agonists or GW501516 known as a PPARδ agonist.

Therefore, it is inferred from the results that the compounds of the present invention are more effective as potent agents for preventing and/or treating arteriosclerosis as compared with conventional agents.

### Example 3

An effect on the secretion of MCP-1 (monocyte chemoattractant protein-1) which is secreted from vascular endothelial cells and causes adhesion and migration of monocytes was investigated using the MCC-555 mentioned in Example 1 as follows.

An effect on the secretion of MCP-1 from vascular endothelial cells was investigated using human aortic endothelial cells (available from Clonetics Corp., USA, and hereienafter abbreviated as "HAEC"). In usual, the HAEC cells were cultured in EGM-2 medium (Clonetics) containing 2% FBS (Clonetics) in a CO₂ incubator (5% CO₂, 37°C). On the first day, the HAEC cells suspended in EGM-2 medium containing 2% FBS were seeded in 96-well plates at 1 × 10⁵ cells/well and then cultured. On the second day, after the cells were washed with PBS, the culture medium was replaced with 200 µL of EGM-2 medium containing 0.4% FBS and the test agent at various concentrations (0.03 to 30 µM) (final DMSO concentration: 0.1%) to culture the cells for 24 hours. On the third day, after the cells were washed with PBS, the culture medium was replaced with 200 µL of EGM-2 medium containing 0.4% FBS, TNF-α (10 ng/mL), and the test agent at various concentrations (0.03 to 30 µM) (final DMSO concentration: 0.1%) to stimulate the cells for 4 hours. After stimulation with TNF-α, the culture medium was collected to measure the MCP-1 concentration of the culture medium by the use of a human MCP-1 ELISA kit (Biosource).

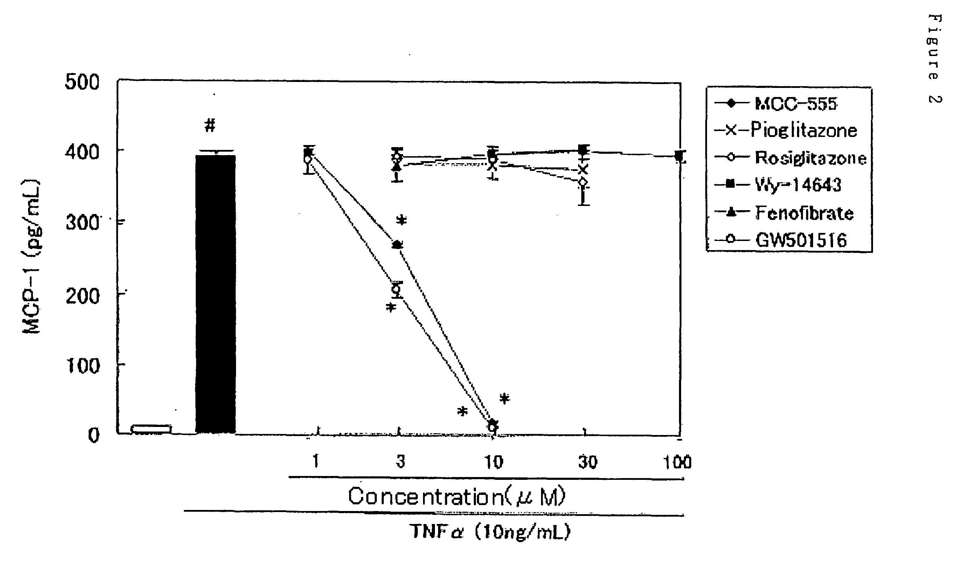

The results are shown in FIG. 2. The data of known compounds having a similar structure (Pioglitazone represented by the formula (II) and Rosiglitazone represented by the formula (III)) are also shown.

As was apparent from the results, although Pioglitazone and Rosiglitazone known as PPARγ agonists, and Fenofibrate and Wy-14643 known as PPARα agonists had no effect on the secretion of MCP-1 induced by stimulation with TNF-α, the compound of the present invention and GW501516 known as a PPARδ agonist suppressed the secretion of MCP-1 induced by stimulation with TNF-α in human vascular endothelial cells.

### INDUSTRIAL APPLICABILITY

According to the present invention, more potent and novel preventive and/or therapeutic medicaments for the diseases attributed to arteriosclerotic activity can be obtained.

The present application was filed with claiming the conventional priority based on Japanese Patent Application No. 2001-252388.

## Claims

1. A preventive and/or therapeutic medicament for diseases attributed to arteriosclerotic activity which comprises as an active ingredient a compound represented by the following formula (I) or a pharmaceutically acceptable salt thereof 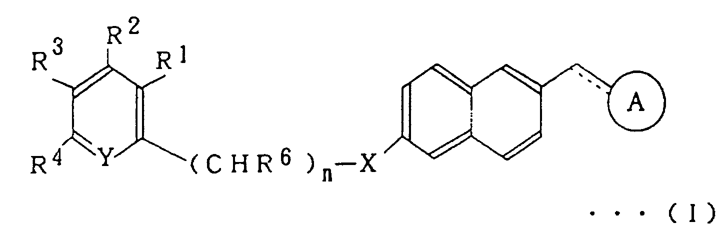 In the above formula, 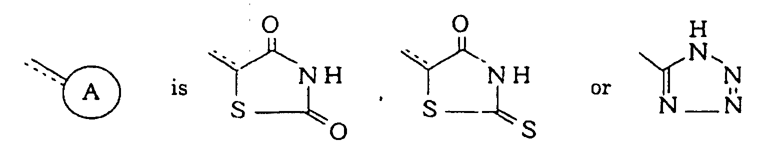 -X- represents -O- or -S-; =Y- represents =N- or =CR⁵-; wherein R¹, R², R³, R⁴ and R⁵ each independently represents hydrogen atom, a halogen atom, an alkyl group, an aryl group, an alkoxy group, an alkoxyalkoxy group, an aryloxy group, alkanoyloxy group, an arylcarbonyloxy group, carboxyl group, an alkoxycarbonyl group, an aryloxycarbonyl group, carbamoyl group, an alkylaminocarbonyl group, an arylaminocarbonyl group, amino group, an alkylamino group, an alkanoylamino group, an arylcarbonylamino group, ethylenedioxymethyl group, formyl group, cyano group, nitro group or a trihalomethyl group; R⁶ represents hydrogen atom, an alkyl group which may be substituted or an aryl group which may be substituted; n is an integer of 0 to 3; and the dotted line indicates that the linkage may be a double bond.

2. The preventive and/or therapeutic medicament according to Claim 1, wherein 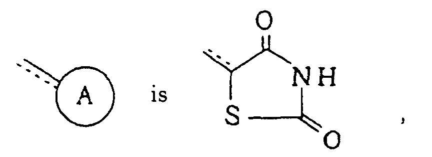 -X- represents -O-; =Y- represents =CR⁵-; R¹, R², R³ and R⁴ each independently represents hydrogen atom or a halogen atom; R⁵ represents hydrogen atom; R⁶ represents hydrogen atom; n is 1; and the dotted line indicates that said linkage is a single bond in the compounds of formula (I) of Claim 1.

3. The preventive and/or therapeutic medicament according to Claim 2, wherein R¹ represents fluorine atom; R², R³ and R⁴ each represents hydrogen atom in the compounds of formula (I) of Claim 1.

4. The preventive and/or therapeutic medicament according to any one of Claims 1 to 3, which comprises exhibiting PPARα, PPARγ, and PPARδ activating activities.

5. The preventive and/or therapeutic medicament according to any one of Claims 1 to 4, wherein the diseases attributed to arteriosclerotic activity are ischemic heart diseases or acute coronary arterial syndromes.

6. A PPARδ activating agent which comprises as an active ingredient the compounds represented by the formula (I) of Claim 1 and pharmaceutically acceptable salts thereof.

7. The activator according to Claim 6, wherein 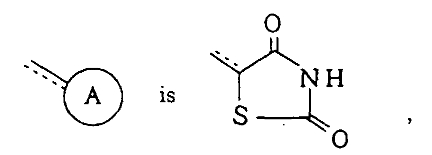 -X- represents -O-; =Y- represents =CR⁵-; R¹, R², R³ and R⁴ each independently represents hydrogen atom or a halogen atom; R⁵ represents hydrogen atom; R⁶ represents hydrogen atom; n is 1; and the dotted line indicates that said linkage is a single bond in the compounds of formula (I) of Claim 1.

8. The activating agent according to Claim 7, wherein R¹ represents fluorine atom; R², R³ and R⁴ each represents hydrogen atom in the compounds of formula (I) of Claim 1.

9. The activating agent according to any one of Claims 6 to 8, which comprises exhibiting PPARα and PPARγ activating activities.

10. An inhibitor for the expression of adhesion molecule at the vascular endothelial cell which comprises as an active ingredient the compounds represented by the formula (I) of Claim 1 and pharmaceutically acceptable salts thereof.

11. The activator according to Claim 10, wherein 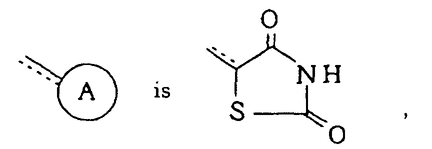 -X- represents -O-; =Y- represents =CR⁵⁻; R¹, R², R³ and R⁴ each independently represents hydrogen atom or a halogen atom; R⁵ represents hydrogen atom; R⁶ represents hydrogen atom; n is 1; and the dotted line indicates that said linkage is a single bond in the compounds of formula (I) of Claim 1.

12. The inhibitor according to Claim 11, wherein R¹ represents fluorine atom; R², R³ and R⁴ each represents hydrogen atom in the compounds of formula (I) of Claim 1.

13. The activator according to any one of Claims 10 to 12, wherein the adhesion molecule is VCAM-1.

14. The inhibitor for the secretion of molecule caused the adhesion and migration of monocyte in vascular endothelial cell which comprises as an active ingredient the compounds represented by the formula (I) of Claim 1 and the pharmaceutically acceptable salts thereof.

15. The inhibitor according to Claim 14, wherein 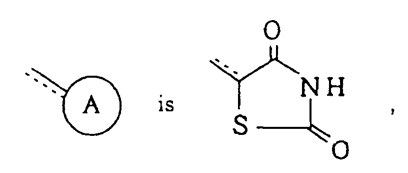 -X- represents -O-; =Y- represents =CR⁵-; R¹, R², R³ and R⁴ each independently represents hydrogen atom or a halogen atom; R⁵ represents hydrogen atom; R⁶ represents hydrogen atom; n is 1; and the dotted line indicates that said linkage is a single bond in the compounds of formula (I) of Claim 1.

16. The inhibitor according to Claim 15, wherein R¹ represents fluorine atom; R², R³ and R⁴ each represents hydrogen atom in the compounds of formula (I) of Claim 1.

17. The inhibitor according to any one of Claims 14 to 16, wherein the molecule caused the adhesion and migration of monocyte is MCP-1.
